# EUROPÄISCHE PATENTSCHRIFT

(11) **EP 1 845 945 B1**
(45) Veröffentlichungstag und Bekanntmachung des Hinweises auf die Patenterteilung: **22.11.2017**
(21) Anmeldenummer: 06706797.5
(22) Anmeldetag: 09.02.2006
(51) Int. Cl.: A61K 9/14, A61K 9/00

(54) **HERSTELLUNG VON DOSIERUNGSFORMEN MIT EINER FESTEN DISPERSION EINES MIKROKRISTALLINEN WIRKSTOFFS**
PROCESS FOR THE PREPARATION OF DOSAGE FORMS COMPRISING A SOLID DISPERSION OF A MICROCRYSTALLINE ACTIVE AGENT
PROCEDÉ POUR LA PRÉPARATION DES FORMES DE DOSAGE COMPRENANT UNE DISPERSION SOLIDE D'UN AGENT ACTIVE MICROCRISTALLINE

(30) Priorität: 11.02.2005 EP 05002955
(43) Veröffentlichungstag der Anmeldung: 24.10.2007
(73) Patentinhaber: AbbVie Deutschland GmbH & Co KG, 65205 Wiesbaden (DE)
(72) Erfinder: ROSENBERG, Jörg, 67158 Ellerstadt (DE); MÄGERLEIN, Markus, 68167 Mannheim (DE); LIEPOLD, Bernd, 69121 Heildelberg (DE); BREITENBACH, Jörg, 68199 Mannheim (DE)
(74) Vertreter: Reitstötter Kinzebach
(86) Internationale Anmeldenummer: PCT/EP2006/001164
(87) Internationale Veröffentlichungsnummer: WO 2006/084696

(56) Entgegenhaltungen:
- EP-A- 0 580 860
- EP-A- 0 988 863
- CH-A5- 670 201
- US-A1- 2003 170 309
- GINÉS: "Thermal investigation of crystallization of poly(ethylene glycol)s in solid dispersions containing oxazepam" INTERNATIONL JOURNAL OF PHARMACEUTICS, Bd. 143, 1996, Seiten 247-253, XP002379985
- SUZUKI H ET AL: "SOME FACTORS INFLUENCING THE DISSOLUTION OF SOLID DISPERSIONS WITH NICOTINAMIDE AND HYDROXYPROPYLMETHYLCELLULOSE AS COMBINED CARRIERS" CHEMICAL AND PHARMACEUTICAL BULLETIN, PHARMACEUTICAL SOCIETY OF JAPAN, TOKYO, JP, Bd. 46, Nr. 6, Juni 1998 (1998-06), Seiten 1015-1020, XP000766598 ISSN: 0009-2363
- PATENT ABSTRACTS OF JAPAN Bd. 2003, Nr. 12, 5. Dezember 2003 (2003-12-05) & JP 2004 267041 A (SANEI GEN FFI INC), 30. September 2004 (2004-09-30)

## Beschreibung

Die vorliegende Erfindung betrifft ein Verfahren zur Herstellung von Dosierungsformen, die eine feste Dispersion eines mikrokristallinen Wirkstoffs umfassen.

Um die Bioverfügbarkeit schwerlöslicher Wirkstoffe zu verbessern, ist es erwünscht, die Oberfläche der Wirkstoffe zu vergrößern, d. h. die Wirkstoffe zu sehr kleinen Partikeln zu zerkleinern. Die bekannten Mahltechniken haben aber verschiedene Nachteile. Es sind sehr lange Mahlzeiten erforderlich, um hinreichend kleine Partikel zu erhalten. Abrieb der verwendeten Mahlkörper und Kontamination des Wirkstoffs mit Rückständen aus der Mühle sind vielfach nicht vermeidbar.

Die Erzeugung kleiner Wirkstoffkristalle durch kontrollierte Kristallisation des Wirkstoffs ist bekannt. Die EP-A 0 156 080 beschreibt die Herstellung einer Wirkstoffzubereitung zur perkutanen Anwendung. Ein Wirkstoff und ein Polymer werden in einem Lösungsmittel gelöst; die Lösung wird auf einem Träger ausgestrichen. Beim Trocknen kristallisiert ein Teil des Wirkstoffs in Form kleiner Partikel aus.

Die DE 35 20 184 offenbart eine galenische Retardform, in der ein Wirkstoff in zusammenhängender kristalliner Form in einer wasserlöslichen kristallinen Matrix enthalten ist. Zur Herstellung löst man den Wirkstoff in geschmolzenem Polyethylenglycol und kühlt die Schmelze ab. Polyethylenglycole weisen typischerweise Glasübergangstemperaturen von weniger als 0 °C auf. Aufgrund der geringen Glasübergangstemperatur zeigen die galenischen Formen eine niedrige Erweichungstemperatur; die galenischen Formen neigen daher zu kaltem Fluss und weisen eine ungenügende Lagerstabilität auf. Außerdem sind viele Wirkstoffe in dem geschmolzenen Polyethylenglycol nur unzureichend löslich. Polyethylenglycole zählen zur Substanzklasse der Ether. Bekanntlich neigen diese insbesondere unter dem Einfluss von Licht, Wärme und Sauerstoff zur Peroxidbildung. Polyethylenglycole sind daher mit oxidationsempfindlichen Wirkstoffen wie z. B. Antibiotika, inkompatibel.

Die WO 93/20138 beschreibt ein zweistufiges Verfahren zum Einbringen einer teilweise löslichen Verbindung in eine Polymermatrix. Eine erste Portion der Verbindung wird in löslicher Phase mit dem Polymer nahe bei der Sättigungskonzentration gemischt; dann wird eine zweite Portion der Verbindung in das Polymer gemischt, so dass sie sich nicht im Polymer auflöst. Eine Abscheidung des gelösten Anteils auf den Partikeln der zweiten Portion soll vermieden werden.

Es wurde nun gefunden, dass man Wirkstoffkristalle geeigneter Größe in kontrollierter Weise aus der Schmelze bestimmter Polymere kristallisieren kann. Die Wirkstoffkristalle werden gleichzeitig in eine Polymermatrix eingeschlossen und stabilisiert.

Die Erfindung betrifft daher ein Verfahren zur Herstellung von Dosierungsformen mit einer festen Dispersion eines mikrokristallinen Wirkstoffs, bei dem man
a) ein thermoplastisches Polymer mit einer Glasübergangstemperatur Tg von wenigstens 40 °C schmilzt und einen Wirkstoff in der Schmelze homogen löst, der sich in 100 ml Wasser bei 22°C zu weniger als 5 g löst,
   wobei das thermoplastische Polymer ausgewählt ist unter Polyvinylpyrrolidon, Copolymerisaten von N-Vinylpyrrolidon und Vinylacetat und/oder Vinylpropionat, Copolymerisaten von Vinylacetat und Crotonsäure, teilverseiftem Polyvinylacetat, Polyvinylalkohol, Polyhydroxyalkylacrylaten, Polyhydroxyalkylmethacrylaten, Polyacrylaten und Polymethacrylaten, Copolymerisaten von Methylmethacrylat und Acrylsäure, Polyethylenglycolen, Alkylcellulosen, Hydroxyalkylcellulosen, Hydroxyalkyl-Alkylcellulosen und Celluloseestern;
b) in der erhaltenen Masse Kristallisation des Wirkstoffs initiiert
   durch Zugabe eines Nichtlösers, wobei der Nichtlöser eine Verbindung ist, worin weniger als 1 g Wirkstoff in 100 ml Nichtlöser bei der Temperatur der Zugabe löslich ist, oder
   durch Zugabe eines Derivatisierungsreagenzes, wobei das Derivatisierungsreagenz unter einer Säure oder einer Base ausgewählt und in der Lage ist, den Wirkstoff in ein Basen- oder Säureadditionssalz umzuwandeln, das in der Masse schlechter löslich ist als der freie Wirkstoff;
c) dann die Masse abkühlt und erstarrt.

Beispielsweise initiiert man im Schritt b) die Kristallisation des Wirkstoffs bei einer Temperatur bei oder oberhalb der Tg des Polymers und kühlt die Masse dann im Schritt c) auf eine Temperatur unterhalb der Tg.

Die Wirkstoff-Mikrokristalle sind in der erhaltenen festen Dispersion in einer Matrix des thermoplastischen Polymers eingebettet und sind auf diese Weise gegen Agglomeration und ähnliche Phänomene stabilisiert. Durch die Gegenwart und innige räumliche Nähe des Polymers und gegebenenfalls von Solubilisatoren ist die Solubilisierung des Wirkstoffs bei der Anwendung der Dosierungsform erleichtert. Dadurch wird insgesamt die Bioverfügbarkeit des Wirkstoffs verbessert.

Die Mikrokristalle weisen typischerweise eine mittlere Teilchengröße (in Richtung der größten Raumausdehnung) von 500 nm bis 100 µm, vorzugsweise von 1 µm bis 80 µm und insbesondere von 5 µm bis 50 µm auf.

Unter Dosierungsformen sind alle Formen zu verstehen, die zur Verwendung als Arzneimittel, insbesondere zur oralen Verabreichung, Pflanzenbehandlungsmittel, Futtermittel und Nahrungsergänzungsmittel geeignet sind. Dazu gehören beispielsweise Tabletten jeglicher Form, Pellets oder Granulate. Hierzu zählen weiterhin Formen wie Folien, Filme, Implantate und Suppositorien.

Unter "Schmelze" wird ein fließfähiges Gemisch verstanden, das in der Lage ist, bei Temperaturerniedrigung aufgrund einer Phasenumwandlung des darin enthaltenen thermoplastischen Polymers in einen festen Zustand überzugehen.

Die "Glasübergangstemperatur" (im Folgenden abgekürzt als "Tg") ist ein wichtiger Parameter zur Kennzeichnung der physikalischen Eigenschaften eines thermoplastischen Polymers. Beim Abkühlen eines verflüssigten Polymer "friert" das Polymer zu einem glasartigen Zustand ein. Der Übergang in den glasartigen Zustand ohne Kristallisation wird als "Glasübergang" bezeichnet. Die Temperatur bei diesem Übergang ist die "Tg". Der Übergang ist im Wesentlichen ein "Einfrieren", d. h. ein Relaxationsvorgang. Bei oder unterhalb der Tg liegt keine Segmentbeweglichkeit mehr vor; es erfolgen keine Mikro-Brownsche Bewegungen. Die Tg kann durch Zugabe von Weichmachern beeinflusst werden. Im Allgemeinen nimmt die Tg mit zunehmender Weichmachermenge ab. Für die Zwecke der vorliegenden Anmeldung wird unter der Glasübergangstemperatur diejenige verstanden, die das Polymer in der Masse aufweist, d. h. unter Berücksichtigung vorhandener Weichmacher und gegebenenfalls weichmachender Effekte weiterer Komponenten

Zur Herstellung der festen Dosierungsformen wird bei einer erhöhten Temperatur, d. h. einer Temperatur bei oder oberhalb des Erweichungspunkts des thermoplastischen Polymers, z. B. im Bereich von 80 bis 200 °C, vorzugsweise 90 bis 180 °C, eine Schmelze, d. h. eine fließfähige kohäsive Masse hergestellt, in der der Wirkstoff gelöst ist. In der Masse wird die Kristallisation des Wirkstoffs initiiert.

Man kann die Kristallisation in der Schmelze initiierten, d. h. in der noch fließfähigen Masse, oder in der nicht mehr fließfähigen aber noch nicht völlig erstarrten Masse. Die Masse wird dann, gegebenenfalls nach einem Formgebungsschnitt, abgekühlt und erstarrt.

Es ist bevorzugt, dass die Kristallisation bei einer Temperatur von wenigstens 35 °C, insbesondere wenigstens 45 °C initiiert wird. In bestimmten Ausführungsformen initiiert man die Kristallisation bei oder oberhalb der Tg des Polymers. Auf diese Weise werden reproduzierbar kleine Partikel weitgehend einheitlicher Partikelgröße erhalten. Das erfindungsgemäße Verfahren unterscheidet sich daher von dem Phänomen der (unerwünschten) Rekristallisation von Wirkstoffen aus molekulardispersen Zubereitungen, die auch als feste Lösungen bezeichnet werden, bei der Lagerung. Bei diesen unkontrollierten Rekristallisationsphänomenen bilden sich Partikel mit sehr heterogener Grö-ßenverteilung.

Die Kristallisation kann auf verschiedene Art initiiert werden, die nachstehend im Einzelnen beschrieben sind. Selbstverständlich können zwei oder mehrere der beschriebenen Maßnahmen kombiniert angewendet werden.

In einer Ausführungsform des erfindungsgemäßen Verfahrens gibt man einen Nichtlöser zu der Masse und initiiert so die Kristallisation des Wirkstoffs. Unter "Nichtlöser" wird eine Verbindung verstanden, in der der Wirkstoff unlöslich oder schlecht löslich ist, d.h. weniger als 1 g Wirkstoff in 100 ml Nichtlöser bei der Temperatur der Zugabe, und die vorzugsweise mit den übrigen Komponenten der Masse vollständig mischbar oder darin löslich ist. Der Nichtlöser ist in der Regel eine Flüssigkeit oder Schmelze und wird vorzugsweise so ausgewählt, dass er physiologisch verträglich ist. Der Nichtlöser kann eine niedermolekulare Verbindung sein, wie Wasser, Alkohole wie Ethanol, n-Propanol, Isopropanol oder n-Butanol, Polyole wie Ethylenglycol, Propylenglycol, Diethylenglycol oder Glycerin, Polyalkylenglycole wie Polyethylenglycole (vorzugsweise mit einem zahlenmittleren Molekulargewicht von weniger als 1000), Lipide wie Triglyceride. Der Nichtlöser wird zweckmäßigerweise mit der Schmelze homogen vermischt, vorzugsweise unter scherenden Bedingungen.

In einer anderen Ausführungsform wandelt man den Wirkstoff durch Zugabe eines unter einer Säure oder einer Base ausgewählten Derivatisierungsreagenzes in ein Basen- oder Säureadditionssalz um, das in der Masse schlechter löslich ist als der freie Wirkstoff. Geeignete Basen oder Säuren sind unter den üblichen physiologisch verträglichen Basen und Säuren ausgewählt. Geeignete Basen sind z. B. Natrium- oder Kaliumcarbonat, Natrium- oder Kaliumhydrogencarbonat, Aminbasen, wie Triethanolamin oder Lysin und dergleichen. Als physiologisch verträgliche organische und anorganische Säuren kommen beispielsweise Salzsäure, Bromwasserstoffsäure, Phosphorsäure, Schwefelsäure, C₁-C₄-Alkylsulfonsäuren wie Methansulfonsäure, aromatische Sulfonsäuren wie Benzolsulfonsäure und Toluolsulfonsäure, Essigsäure, Citronensäure, Äpfelsäure, Bernsteinsäure, Asparaginsäure, Glutaminsäure, Crotonsäure, Glycolsäure, Acetylsalicylsäure, Oxalsäure, Maleinsäure, Fumarsäure, Milchsäure, Weinsäure, Adipinsäure und Benzoesäure in Betracht.

Andere Derivatisierungsreagenzien, die in Betracht kommen, sind Komplexbildner wie Cyclodextrine.

Das Derivatisierungsreagenz wird bevorzugt in flüssiger oder gelöster Form zu der Masse gegeben.

Man kann Impfkristalle des Wirkstoffs zu der Masse geben und so die Kristallisation des Wirkstoffs initiieren. Die Temperatur, bei der sich die Masse bei der Zugabe der Impfkristalle befindet, wird dabei zweckmäßigerweise so gewählt, dass keine nennenswerte Auflösung der Impfkristalle erfolgt, d. h. bei der gewählten Temperatur soll die Masse bezüglich des Wirkstoffs im Wesentlichen gesättigt oder übersättigt sein. Die Impfkristalle werden vorzugsweise als Suspension in einem geeigneten Suspensionsmedium zugegeben. Ein Verfahren bei dem man die Kristallisation des Wirkstoffs ausschließlich durch die Zugabe der Impfkristalle initiiert, ist nicht erfindungsgemäß.

Man kann die Kristallisation des Wirkstoffs initiieren, indem man die Schmelze eine ausreichende Zeit bei einer Temperatur hält, die geringer ist als die Temperatur, bei der der Wirkstoff vollständig in der Schmelze löslich ist und die vorzugsweise wenigstens 35 °C, insbesondere wenigstens 45 °C beträgt. Man nutzt eine temperaturabhängige Löslichkeit des Wirkstoffs in der Masse. Beim kontrollierten Abkühlen der Masse kristallisiert der Wirkstoff aus der Masse aus. Ein Verfahren bei dem man die Kristallisation des Wirkstoffs ausschließlich dadurch initiiert, dass man die Schmelze eine ausreichende Zeit bei einer Temperatur hält, die geringer ist als die Temperatur, bei der der Wirkstoff vollständig in der Schmelze löslich ist, ist nicht erfindungsgemäß.

Das Auskristallisieren des Wirkstoffs kann mit einer Umwandlung einer ursprünglich eingesetzten polymorphen Form des Wirkstoffs in eine andere polymorphe Form des Wirkstoffs einher gehen.

Als thermoplastische Polymere für das erfindungsgemäße Verfahren kommen physiologisch verträgliche, wasserlösliche oder wasserdispergierbare Polymere mit einer Tg von wenigstens 40 °C, vorzugsweise 50 bis 180 °C, in Betracht, die sich unzersetzt oder ohne nennenswerte Zersetzung schmelzen lassen. Geeignet sind auch Polymere, die unter Zusatz geeigneter Weichmacher schmelzbar werden.

Erfindungsgemäß geeignete thermoplastische Polymere sind
Polyvinylpyrrolidon (PVP),
Copolymerisate von N-Vinylpyrrolidon und Vinylacetat und/oder Vinylpropionat, Copolymerisate von Vinylacetat und Crotonsäure,
teilverseiftes Polyvinylacetat, Polyvinylalkohol,
Polyhydroxyalkylacrylate, Polyhydroxyalkylmethacrylate,
Polyacrylate und Polymethacrylate (Eudragit-Typen),
Copolymerisate von Methylmethacrylat und Acrylsäure,
Polyethylenglycole,
Alkylcellulosen, insbesondere Methylcellulose und Ethylcellulose, Hydroxyalkylcellulosen, insbesondere Hydroxypropylcellulose (HPC), Hydroxyalkyl-Alkylcellulosen, insbesondere Hydroxypropylmethylcellulose (HPMC), Celluloseester wie Cellulosephthalate, insbesondere Celluloseacetatphthalat, Hydroxypropylmethylcellulosephthalat und Hydroxypropylmethylcelluloseacetatsuccinat (HPM-CAS).

Davon sind Homo- oder Copolymere von Vinylpyrrolidon besonders bevorzugt, z. B. Polyvinylpyrrolidon mit K-Werten nach Fikentscher von 12 bis 100, vorzugsweise 17 bis 30, oder Copolymere von 30 bis 70 Gew.-% N-Vinylpyrrolidon (VP) und 70 bis 30 Gew.-% Vinylacetat (VA), wie z. B. ein Copolymer aus 60 Gew.-% VP und 40 Gew.-% VA. Besonders bevorzugt sind auch Hydroxypropylcellulose und Hydroxypropylmethylcellulose.

Selbstverständlich können auch Gemische der genannten Polymere eingesetzt werden. Die Löslichkeit des Wirkstoffs in der Schmelze soll bevorzugt mehr als 20 Gew.-%, insbesondere mehr als 40 Gew.-%, bezogen auf die Summe von Wirkstoff, Polymer und fakultative Komponenten, betragen.

Unter Wirkstoffen im Sinne der Erfindung sind alle Stoffe mit einer erwünschten physiologischen Wirkung auf den menschlichen oder tierischen Körper oder Pflanzen zu verstehen. Es handelt sich insbesondere um pharmazeutische Wirkstoffe. Die Wirkstoffmenge pro Dosiseinheit kann in weiten Grenzen variieren. Sie wird in der Regel so gewählt, dass sie zur Erzielung der gewünschten Wirkung ausreicht. Auch WirkstoffKombinationen können eingesetzt werden.

Der Wirkstoff ist unlöslich oder schwer löslich in Wasser; weniger als 5 g Wirkstoff, insbesondere weniger als 1 g Wirkstoff, lösen sich in 100 ml Wasser bei 22 °C.

Beispiele derartiger unlöslicher oder schwer löslicher Verbindungen sind Analgetika und Entzündungshemmer wie Fentanyl, Indomethacin, Ketoprofen, Nabumeton, Oxyphenbutazon, Paracetamol, Phenylbutazone, Piroxicam, Tramadol; Antiarrhythmika, wie Gallopamil, Procainamid, Quinidin, Verapamil;
Antiinfektiva wie Amoxicillin, Ampicillin, Benzathin, Penicillin, Benzylpenicillin, Cefaclor, Cefadroxil, Cefprozil, Cefuroximaxetil, Cephalexin, Chloramphenicol, Chloroquin, Ciprofloxacin, Clarithromycin, Clavulansäure, Clindamycin, Doxyxyclin, Erythromycin, Flucloxacillin, Halofantrin, Isoniazid, Kanamycin, Lincomycin, Mefloquin, Minocyclin, Nafcillin, Neomycin, Norfloxacin, Ofloxacin, Oxacillin, Phenoxymethyl-Penicillin, Pyrimethamin-sulfadoxime, Chinin, Streptomycin; Antikoagulantien wie Warfarin;
Antidepressiva wie Amitriptylin, Amoxapin, Atibeprone, Butriptylin, Clomipramin, Desipramin, Dothiepin, Doxepin, Fluoxetin, Fluvoxamine, Gepiron, Imipramin, Mianserin, Milnacipran, Nortriptylin, Paroxetin, Sertralin;
Antidiabetica wie Glibenclamid, Metformin;
Antiepileptica wie Carbamazepin, Clonazepam, Ethosuximid, Phenobarbiton, Phenytoin, Primidon, Topiramat, Valpromid;
Antimycotica wie Amphotericin, Clotrimazol, Econazol, Fluconazol, Flucytosin, Griseofulvin, Itraconazol, Ketoconazol, Miconazolnitrat, Nystatin, Terbinafin, Voriconazol;
Gichtmittel wie Benzbromaron, Probenecid;
Antihistaminica wie Astemizol, Cinnarizin, Cyproheptadin, Decarboethoxyloratadin, Fexofenadin, Flunarizin, Levocabastin, Loratadin, Norastemizol, Oxatomid, Promethazin, Terfenadin;
Antihypertensiva wie Captopril, Clonidin, Cyclizin, Diazoxid, Dihydralazin, Enalapril, Fosinopril, Guanethidin, Ketanserin, Lisinopril, Minoxidil, Prazosin, Ramipril, Rescinnamine, Reserpine, Terazosin;
Muskarinantagonisten wie Atropinsulfate, Hyoscin;
Virostatica wie Acyclovir, AZT, ddC, ddl, Ganciclovir, Lovirid, Tivirapin, 3TC, Delavirdin, Indinavir, Nelfinavir, Ritonavir, Saquinavir, Lopinavir;
Zytostatica und Antimetaboliten wie Adriamycin, Cladribin, Dactinomycin, Daunorubicin, Doxorubicin, Etoposid, Mitomycm, Mitoxantron, Paclitaxel, Taxol, Taxoter, Trimetrexat, Vincristin, Vinblastin;
Migränemittel wie Alniditan, Naratriptan, Sumatriptan;
Parkinsonmittel wie Bromocryptinmesylat, Carbidopa, Levodopa, Selegilin;
Antipsychotica, Hypnotica, Anxiolytica und Sedativa wie Alprazolam, Buspiron, Chlordiazepoxid, Chlorpromazin, Chlorprothixen, Clozapin, Diazepam, Flupenthixol, Fluphenazin, Flurazepam, Haloperidol, 9-Hydroxyrisperidon, Lorazepam, Mazapertin, Melperon, Methaqualon, Olanzapin, Oxazepam, Pimozid, Pipamperon, Piracetam, Promazin, Risperidon, Selfotel, Seroquel, Sertindol, Sulpirid, Temazepam, Thioridazin, Thiothixen, Triazolam, Trifluoperazin, Trifluperidol, Triflupromazin, Ziprasidon, Zolpidem;
Neuroprotektiva wie Lubeluzol, Lubeluzoloxid, Riluzol, Aptiganel, Eliprodil, Remacemid;
Antitussiva wie Dextromethorphan, Lävodropropizin, Noscapin;
Beta-Blocker wie Atenolol, Bupranolol, Carvedilol, Labetalol, Metipranolol, Metoprolol, Nebivolol, Oxprenolol, Propanolol;
Inotropica wie Amrinon, Digitoxin, Digoxin, Milrinon;
Corticosteroide wie Beclomethason-dipropionat, Betamethason, Budesonid, Cortison, Dexamethason, Fludrocortison, Hydrocortison, Methylprednisolon, Paramethason, Prednisolon, Prednison, Triamcinolon;
Antiseptica wie Chlorhexidin;
Diuretica wie Acetazolamid, Amilorid, Benzthiazid, Chlorothiazid, Chlorthalidon, Dichlorphenamid, Ethacrynsäure, Ethoxzolamid, Frusemid, Hydrochlorthiazid, Hydroflumethiazid, Isosorbid, Polythiazid, Spironolacton, Triamteren, Trichlormethiazid;
Ergotalkaloide wie Codergocrin, Ergotamin, Nicergolin;
Magen-Darm-Mittel wie Bromoprid, Cimetidin, Cisaprid, Cleboprid, Diphenoxylat, Domperidon, Famotidin, Lansoprazol, Loperamid, Loperamidoxid, Mesalazin, Metoclopramid, Mosaprid, Nizatidin, Norcisaprid, Olsalazin, Omeprazol, Pantoprazol, Perprazol, Pirenzepin, Prucaloprid, Ranitidin, Rabeprazol, Ridogrel, Sulphasalazin;
Hämostatica wie Aminocapronsäure;
Immunosuppressiva wie Cyclosporin A, Tacrolimus;
Lipidsenker wie Atorvastatin, Lovastatin, Pravastatin, Probucol, Simvastatin, Fenofibrinsäure, Fenofibrat;
Lokalanästhetica wie Benzocain, Lignocain;
Opioidanalgetica wie Buprenorphin, Codein, Dextromoramid, Dextropropoxyphen, Dihydrocodein, Hydrocodon, Oxycodon, Morphin, Papaverin, Pentazocin, Pethidin;
Parasympathomimetica wie Eptastigmin, Galanthamin, Metrifonat, Neöstigmin, Physostigmin, Tacrin, Donepezil, Rivastigmin, Milamelin, Sabcomelin, Talsaclidin, Xanomelin, Memantin, Lazabemid;
Hormone, z. B. Androgene wie Methyltestosteron, Oxymetholon, Stanozolol; Östrogene wie konjugierte Östrogene, Ethinylöstradiol, Mestranol, Östradiol, Östriol, Östron; Progestogene; Chlormadinonacetat, Cyproteronacetat, 17-Deacetylnorgestimat, Desogestrel, Dienogest, Dydrogesteron, Ethynodioldiacetat, Gestoden, 3-Ketodesogestrel, Levonorgestrel, Lynestrenol, Medroxy-progesteronacetat, Megestrol, Norethindron, Norethindronacetat, Norethisteron, Norethisteronacetat, Norethynodrel, Norgestimat, Norgestrel, Norgestrienon, Progesteron, Quingestanolacetat;
Stimulantien wie Sildenafil;
Sympathomimetica wie Ephedrin, Clenbuterol, Fenoterol, Norfenefrin, Pseudoephedrin;
Vasodilatatoren wie Amlodipin, Buflomedil, Buphenin, Carbocromen, Diltiazem, Dipyridamol, Isosorbiddinitrat, Lidoflazin, Molsidomin, Nicardipin, Nifedipin, Nimodipin, Oxpentifyllin.

Die Masse kann daneben verschiedene fakultative Hilfsstoffe umfassen. Derartige fakultative Hilfsstoffe sind:
Weichmacher wie z.B. C₇-C₃₀-Alkanole, Ethylenglycol, Propylenglycol, Glycerin, Trimethylolpropan, Triethylenglycol, Butandiole, Pentanole, wie Pentaerythrit und Hexanole, Polyalkylenglycole, vorzugsweise mit einem Molekulargewicht von 200 bis 1000, wie z.B. Polyethylenglycole, Polypropylenglycole und Polyethylenpropylenglycole, Silicone, aromatische Carbonsäureester (z.B. Dialkylphthalate, Trimellithsäureester, Benzoesäureester, Terephthalsäureester) oder aliphatische Dicarbonsäureester (z.B. Dialkyladipate, Sebacinsäureester, Azelainsäureester, Zitronen- und Weinsäureester), Fettsäureester, wie Glycerinmono-, Glycerindi- oder Glycerintriacetat oder Natriumdiethylsulfosuccinat. Die Konzentration an Weichmacher beträgt soweit vorhanden im Allgemeinen 0,5 bis 30, vorzugsweise 0,5 bis 10 Gew.-%, bezogen auf das Gesamtgewicht von Polymer und Weichmacher. Die Menge an Weichmacher beträgt vorteilhafterweise höchstens 30 Gew.-%, bezogen auf das Gesamtgewicht von Polymer und Weichmacher, damit - im Bereich fester Formen - lagerstabile Formulierungen und Dosierungsformen gebildet werden, die keinen kalten Fluss zeigen.

Zuckeralkohole wie Sorbit, Xylit, Mannit, Maltitol; oder Zuckeralkoholderivate wie Isomalt oder hydrierte kondensierte Palatinose wie in der DE 102 62 005 beschrieben.

Solubilisatoren, wie Sorbitanfettsäureester, polyalkoxylierte Fettsäureester, wie z. B polyalkoxylierte Glyceride, polyalkoxylierte Sorbitanfettsäureester oder Fettsäureester von Polyalkylenglycolen; oder polyalkoxylierte Ether von Fettalkoholen. Eine Fettsäurekette in diesen Verbindungen umfasst in der Regel 8 bis 22 Kohlenstoffatome. Die Polyalkylenoxid-Blöcke umfassen pro Molekül durchschnittlich 4 bis 50 Alkylenoxideinheiten, vorzugsweise Ethylenoxideinheiten.

Geeignete Sorbitanfettsäureester sind Sorbitanmonolaurat, Sorbitanmonopalmitat, Sorbitanmonostearat, Sorbitanmonooleat, Sorbitantristearat, Sorbitantrioleat, Sorbitanmonostearat, Sorbitanmonolaurat oder Sorbitanmonooleat.

Geeignete polyalkoxylierte Sorbitanfettsäurester sind beispielsweise Polyoxyethylen(20)sorbitanmonolaurat, Polyoxyethylen(20)sorbitanmonopalmitat, Polyoxyethylen(20)sorbitanmonostearat, Polyoxyethylen(20)sorbitanmonooleat, Polyoxyethylen(20)sorbitantristearat, Polyoxyethylen(20)sorbitantrioleat, Polyoxyethylen(4)sorbitanmonostearat, Polyoxyethylen(4)sorbitanmonolaurat oder Polyoxyethylen(4)sorbitanmonooleat.

Geeignete polyalkoxylierte Glyceride werden z. B. durch Alkoxylierung von natürlichen oder hydrierten Glyceriden bzw. durch Umesterung von natürlichen oder hydrierten Glyceriden mit Polyalkylenglycolen erhalten. Handelsübliche Beispiele sind Polyoxyethylenglycerolricinoleat-35, Polyoxyethylenglyceroltrihydroxystearat-40 (Cremophor® RH40, BASF AG) sowie polyalkoxylierte Glyceride wie sie unter den Handelsnamen Gelucire® und Labrafil® von Gattefosse erhältlich sind, z. B. Gelucire® 44/14 (Lauroyl-Macrogol-32-glyceride, hergestellt durch Umesterung von hydriertem Palmkemöl mit PEG 1500), Gelucire® 50/13 (Stearoyl-Macrogol-32-glyceride, hergestellt durch Umesterung von hydriertem Palmöl mit PEG 1500) oder Labrafil M1944 CS (Oleoyl-Macrogol-6-glyceride, hergestellt durch Umesterung von Aprikosenkernöl mit PEG 300).

Ein geeigneter Fettsäureester von Polyalkylenglycolen ist z. B. PEG-660-Hydroxystearinsäure (Polyglycolester der 12-Hydroxystearinsäure (70 mol-%) mit 30 mol-% Ethylenglycol).

Geeignete polyalkoxylierte Ether von Fettalkoholen sind z. B. Macrogol-6-Cetylstearylether oder Macrogol-25-Cetylstearylether

Solubilisatoren werden typischerweise in einer Menge von 0,1 bis 15 Gew.-%, vorzugsweise 0,5 bis 10 Gew.-% in der Pulvermischung mitverwendet.

Sprengmittel, wie vernetztes Polyvinylpyrrolidon und vernetzte Natriumcarboxymethylcellulose.

Streckmittel bzw. Füllstoffe, wie Lactose, Cellulose, Silikate oder Kieselsäure,
Schmiermittel, wie Magnesium- und Calciumstearat, Natriumstearylfumarat,
Farbstoffe, wie Azofarbstoffe, organische oder anorganische Pigmente oder Farbstoffe natürlicher Herkunft,
Stabilisatoren, wie Antioxidanzien, Lichtstabilisatoren, Hydroperoxid-Vernichter, Radikalfänger, Stabilisatoren gegen mikrobiellen Befall.

Typischerweise enthält die Masse
10 bis 90 Gew.-%, vorzugsweise 30 bis 80 Gew.-%, thermoplastisches Polymer,
0,5 bis 80 Gew.-%, vorzugsweise 10 bis 60 Gew.-%, Wirkstoff, und
0 bis 40 Gew.-%, vorzugsweise 0 bis 30 Gew.-% fakultative Hilfsstoffe.

Zweckmäßigerweise mischt man die Komponenten oder einen Teil der Komponenten der Schmelze vor dem Erwärmen zu einer Pulvermischung. Das Vermischen der Komponenten zur Pulvermischung erfolgt in üblichen Mischern, wie Pflugscharmischern, Schüttel- bzw. Freifallmischern und dergleichen.

Das Erwärmen der Pulvermischung erfolgt in einer für diesen Zweck üblichen Vorrichtung. Besonders geeignet sind beheizbare Extruder oder Kneter, wie Misch-Knetreaktoren (z. B. ORP, CRP, AP, DTB der Firma List oder Reactotherm der Firma Krauss-Maffei oder Ko-Kneter der Fa. Buss), Doppelmuldenkneter (Trogmischer) und Stempelkneter (Innenmischer) oder Rotor/Stator-Systeme (z. B. Dispax der Firma IKA). Die Verweildauer der Masse im Extruder beträgt vorzugsweise weniger als 5 Minuten, insbesondere weniger als 3 Minuten.

Als Extruder kann man Einschneckenmaschinen, kämmende Schneckenmaschinen oder auch Mehrwellextruder, insbesondere Zweischnecken-Extruder, gleichsinnig oder gegensinnig drehend und gegebenenfalls mit Knetscheiben ausgerüstet, einsetzen. Besonders bevorzugt sind gleichsinnig drehende Doppelschneckenextruder.

Das Beschicken des Extruders bzw. Kneters erfolgt je nach deren Konzeption kontinuierlich oder diskontinuierlich in üblicher Weise. Die Pulvermischung wird vorzugsweise im freien Zulauf, z. B. über eine Differentialdosierwaage eingeführt.

Die Verwendung kontinuierlich arbeitender Kneter bzw. Extruder ist bevorzugt. Dabei führt man das pulverförmiges Gemisch des Polymers und des Wirkstoffs an einem Eintrittsende in ein längliches Extrudergehäuse ein; erwärmt das Gemisch, um eine Schmelze zu erhalten; bewegt die Schmelze durch das Extrudergehäuse zu einem Austrittsende des Extrudergehäuses; und erzeugt einen ausreichenden Gegendruck im Extrudergehäuse, damit die Schmelze aus einem Austrittsende des Extrudergehäuses als ein zusammenhängendes Extrudat austritt.

Das Extrudergehäuse und die Schnecken sind in der Regel in Segmente aufgeteilt. Gehäuse und Schnecken lassen sich daher beliebig zu einer Plastifiziereinheit mit Rohstoffzuführung, Entlüftung oder Entgasung kombinieren. Die Schneckengeometrie kann der zu verarbeitenden Masse angepasst werden, indem die Reihenfolge von Schnecken-, Knet- und Mischelementen geeignet gewählt wird. Die Segmente des Extrudergehäuses lassen sich separat temperieren. Die Beheizung erfolgt z. B. durch Widerstandheizbänder oder durch ein im Mantel zirkulierendes Heizmedium.

Gemäß den vorstehend genau beschriebenen Ausführungsformen führt man an einer zum Austrittsende gelegenen Stelle des Extrudergehäuses den Nichtlöser; oder das Derivatisierungsreagenz in das Extrudergehäuse ein. Die Zugabe des Nichtlösers bzw. des Derivatisierungsreagenzes erfolgt in geeigneter Weise, so dass ein gleichmäßiges Einmischen in die Schmelze erreicht wird. Dies kann der Fachmann z. B. durch Wahl des Zugabeortes und der Schneckengeometrie erreichen.

Die erhaltene Masse wird in der Regel einer Formgebung unterzogen. Dabei kann eine Vielzahl von Formen, je nach Werkzeug und Art der Formung, erzeugt werden. Beispielsweise lässt sich bei Verwendung eines Extruders der extrudierte Strang zwischen einem Band und einer Walze, zwischen zwei Bändern oder zwischen zwei Walzen, wie in der EP-A-358 105 beschrieben, oder durch Kalandrierung in einem Kalander mit zwei Formwalzen, siehe beispielsweise EP-A-240 904, formen. Durch Extrusion und Heiß- oder Kaltabschlag des Stranges können beispielsweise kleinteilige Granulate erhalten werden.

Die erkalteten Massen können anschließend auch zu Pulver gemahlen und dann in üblicher Weise zu Tabletten verpresst werden. Hierbei können Tablettierhilfsmittel wie kolloidale Kieselsäure, Calciumhydrogenphosphat, Lactose, mikrokristalline Cellulose, Stärke oder Magnesiumstearat mitverwendet werden.

Die Erfindung wird durch die nachstehenden Beispiele näher veranschaulicht.

### Beispiel 1

Eine Pulvermischung aus 30 Gew.-% Fenofibrat und 70 Gew.-% Copovidon (Kollidon VA-64, BASF AG Ludwigshafen, Deutschland) wurde in einem Kneter bei einer Temperatur von 100 °C zu einer homogenen transparenten honigartigen Schmelze verarbeitet. In die homogene klare Schmelze wurden 20 Gew.-% Wasser gegeben, wodurch sich die Schmelze spontan eintrübte. Nach dem Abkühlen konnten in den erkalteten trüben Schmelzestücken im Polarisationsmikroskop Kristallnadeln mit einer Länge von 20-50 µm festgestellt werden. Eine Analyse der Schmelzeprobe mittels DSC bestätigte das Vorliegen von kristallinem Wirkstoff.

### Beispiel 2 (Vergleichsbeispiel)

Beispiel 1 wurde wiederholt, wobei jedoch kein Wasser zugesetzt wurde. Die klare Schmelze blieb auch nach dem Abkühlen klar, es wurde keine Rekristallisation beobachtet. Eine Analyse der Schmelzeprobe mittels DSC ergab, dass der Wirkstoff vollständig in nichtkristalliner Form vorlag.

### Beispiel 3

Eine Pulvermischung aus 20 Gew.-% Fenofibrat, 5 Gew.-5 Labrafil M 1944 CS (Oleyl-macrogol-6-glycerid, F. Gattefosse, Frankreich) und 75 Gew.-% Copovidon (Kollidon VA-64, BASF AG Ludwigshafen, Deutschland) wurde in einem Kneter bei einer Temperatur von 100 °C zu einer homogenen transparenten honigartigen Schmelze verarbeitet. In die homogene klare Schmelze wurden 20 Gew.-% Wasser gegeben, wodurch sich die Schmelze spontan eintrübte. Nach dem Abkühlen konnten in den erkalteten trüben Schmelzestücken im Polarisationsmikroskop Kristallnadeln mit einer Größe von weniger als 10 µm festgestellt werden. Eine Analyse der Schmelzeprobe mittels DSC bestätigte das Vorliegen von kristallinem Wirkstoff.

### Beispiel 4

Eine Pulvermischung aus 20 Gew.-% Fenofibrat, 5 Gew.-% Tween 20 (Polyoxyethylen-20-Sorbitan-monolaurat) und 75 Gew.-% Copovidon (Kollidon VA-64, BASF AG Ludwigshafen, Deutschland) wurde in einem Kneter bei einer Temperatur von 100 °C zu einer homogenen transparenten honigartigen Schmelze verarbeitet. In die homogene klare Schmelze wurden 20 Gew.-% Wasser gegeben, wodurch sich die Schmelze spontan eintrübte. Nach dem Abkühlen konnten in den erkalteten trüben Schmelzestücken im Polarisationsmikroskop Kristallnadeln mit einer Länge von 10-20 µm festgestellt werden. Eine Analyse der Schmelzeprobe mittels DSC bestätigte das Vorliegen von kristallinem Wirkstoff.

### Beispiel 5 (Vergleichsversuch)

Man verwendete Atibeprone als Wirkstoff. Das Wirkstoff-Ausgangsmaterial zeigte einen Schmelzpeak in der Differential-Thermoanalyse (DSC) bei 123 °C.

Eine Pulvermischung aus 20 Gew.-% Atibeprone und 80 Gew.-% Hydroxypropylcellulose (Klucel EF, Aqualon, Deutschland) wurde in einem Zweischneckenextruder bei einer Temperatur von 140 °C extrudiert. Aus der Extruderdüse trat ein klarer transparenter Schmelzestrang aus, der an der Luft im noch plastischen Zustand bereits stark eintrübte. In den trüben, erkalteten Strangstücken konnten mittels Polarisationsmikroskopie kleine längliche Kristallnadeln festgestellt werden.

Bei der Analytik mittels DSC zeigte sich, dass der Wirkstoff im erkalteten Extrudat in zwei unterschiedlichen kristallinen polymorphen Formen vorlag. Neben der bereits im Ausgangsmaterial vorhandenen Kristall-Form mit dem Schmelzpunkt 127 °C trat ein weiterer Schmelzpeak einer zweiten kristallinen Form bei 108 °C auf; beide Kristallformen lagen etwa im Verhältnis 1:1 vor.

### Beispiel 6 (Vergleichsversuch)

Der Versuch erfolgte wie in Beispiel 5 angegeben, nur wurde die aus dem Extruder austretende Schmelze durch eine schmale Breitschlitz-Düse ausgetragen, wodurch eine dünne Folie (etwa 0,3 mm Dicke) aus dem Schmelzematerial entstand. Durch die im Verhältnis zur Masse sehr große Oberfläche kühlte diese Folien sehr schnell auf Raumtemperatur ab. Eine Eintrübung der klaren Schmelze durch Rekristallisation des Wirkstoffs in der Schmelze wie in Beispiel 5 erfolgte nicht; die Folie blieb auch nach dem Aushärten der Schmelze klar transparent.

### Beispiel 7 (Vergleichsversuch)

Der Versuch erfolgte wie in Beispiel 6, aber die aus der Extruder-Breitschlitzdüse austretende Folie wurde direkt nach Austrag für wenige Minuten bei einer Temperatur von 80 °C gehalten. Die Folie trübte stark ein und diese Trübung blieb auch anschliessendem Abkühlen auf Raumtemperatur erhalten.

### Beispiel 8

Zu einer homogenen klaren Schmelze bestehend aus 50 Gew.-% Ibuprofen und 50 Gew.-% Copovidon (Kollidon® VA-64, BASF, Deutschland) wurde bei einer Temperatur von 90 °C eine homogene, klare Schmelze erzeugt. Diese Schmelze wurde 2 Minuten weiter geknetet und dann zu dieser Schmelze unter weiterem Kneten fein gepulvertes Natriumcarbonat portionsweise zugegeben (Molverhältnis Ibuprofen : Natriumcarbonat = 2 : 1). Es kam dabei zu einer Gasentwicklung und die Schmelze trübte stark ein. Nach vollständiger Zugabe des Natriumcarbonats wurde noch 3 Minuten bei 90 °C weitergeknetet und dann auf Raumtemperatur ohne weiteres Kneten abgekühlt. In der erkalteten Schmelze konnte kristallines Natrium-Ibuprofenat nachgewiesen werden (WAXS).

## Patentansprüche

1. Verfahren zur Herstellung von Dosierungsformen, die eine feste Dispersion eines mikrokristallinen Wirkstoffs umfassen, bei dem man
a) ein thermoplastisches Polymer mit einer Glasübergangstemperatur Tg von wenigstens 40 °C schmilzt und einen Wirkstoff in der Schmelze homogen löst, der sich in 100 ml Wasser bei 22°C zu weniger als 5 g löst,
wobei das thermoplastische Polymer ausgewählt ist unter Polyvinylpyrrolidon, Copolymerisaten von N-Vinylpyrrolidon und Vinylacetat und/oder Vinylpropionat, Copolymerisaten von Vinylacetat und Crotonsäure, teilverseiftem Polyvinylacetat, Polyvinylalkohol, Polyhydroxyalkylacrylaten, Polyhydroxyalkylmethacrylaten, Polyacrylaten und Polymethacrylaten, Copolymerisaten von Methylmethacrylat und Acrylsäure, Polyethylenglycolen, Alkylcellulosen, Hydroxyalkylcellulosen, Hydroxyalkyl-Alkylcellulosen und Celluloseestern ;
b) in der erhaltenen Masse Kristallisation des Wirkstoffs initiiert
durch Zugabe eines Nichtlösers, wobei der Nichtlöser eine Verbindung ist, worin weniger als 1 g Wirkstoff in 100 ml Nichtlöser bei der Temperatur der Zugabe löslich ist, oder
durch Zugabe eines Derivatisierungsreagenzes, wobei das Derivatisierungsreagenz unter einer Säure oder einer Base ausgewählt und in der Lage ist, den Wirkstoff in ein Basen- oder Säureadditionssalz umzuwandeln, das in der Masse schlechter löslich ist als der freie Wirkstoff;
c) dann die Masse abkühlt und erstarrt.

2. Verfahren nach Anspruch 1, wobei die Alkylcellulose unter Methylcellulose und Ethylcellulose, die Hydroxyalkylcellulose unter Hydroxypropylcellulose, die Hydroxyalkyl-Alkylcellulose unter Hydroxypropylmethylcellulose, der Celluloseester unter Cellulosephthalate, Celluloseacetatphthalat, Hydroxypropylmethylcellulosephthalat und Hydroxypropylmethylcelluloseacetatsuccinat ausgewählt ist.

3. Verfahren nach Anspruch 1, wobei das thermoplastische Polymer unter Polyvinylpyrrolidon mit K-Werten nach Fikentscher von 12 bis 100, und Copolymeren von 30 bis 70 Gew.-% N-Vinylpyrrolidon und 70 bis 30 Gew.-% Vinylacetat ausgewählt ist.

4. Verfahren nach einem der vorhergehenden Ansprüche, wobei der Nichtlöser unter Wasser, Alkoholen, Polyolen und Lipiden ausgewählt ist.

5. Verfahren nach einem der vorhergehenden Ansprüche, wobei die Masse wenigstens einen Weichmacher umfasst.

6. Verfahren nach Anspruch 1, bei dem man
a) ein pulverförmiges Gemisch des Polymers und des Wirkstoffs an einem Eintrittsende in ein längliches Extrudergehäuse einführt;
b) das Gemisch im Extrudergehäuse erwärmt, um eine Schmelze zu erhalten;
c) die Schmelze durch das Extrudergehäuse zu einem Austrittsende des Extrudergehäuses bewegt;
d) einen ausreichenden Gegendruck im Extrudergehäuse erzeugt, damit die Schmelze aus dem Austrittsende des Extrudergehäuses als ein zusammenhängendes Extrudat austritt; und
e) eine Kristallisation des Wirkstoffs initiiert, indem man an einer zum Austrittsende gelegenen Stelle des Extrudergehäuses den Nichtlöser oder das Derivatisierungsreagenz in das Extrudergehäuse einführt und mit der Schmelze mischt.

## Claims

1. Process for producing dosage forms comprising a solid dispersion of a microcrystalline active ingredient, in which
a) a thermoplastic polymer having a glass transition temperature Tg of at least 40°C is melted and an active ingredient is dissolved homogeneously in the melt, which active ingredient dissolves to an extent of less than 5 g in 100 ml of water at 22°C,
the thermoplastic polymer being selected from polyvinylpyrrolidone, copolymers of N-vinylpyrrolidone and vinyl acetate and/or vinyl propionate, copolymers of vinyl acetate and crotonic acid, partially hydrolysed polyvinyl acetate, polyvinyl alcohol, polyhydroxyalkyl acrylates, polyhydroxyalkyl methacrylates, polyacrylates and polymethacrylates, copolymers of methyl methacrylate and acrylic acid, polyethylene glycols, alkylcelluloses, hydroxyalkylcelluloses, hydroxyalkylalkylcelluoses and cellulose esters;
b) crystallization of the active ingredient is initiated in the composition obtained
by addition of a non-solvent, the non-solvent being a compound in which less than 1 g of active ingredient is soluble in 100 ml of non-solvent at the temperature of addition, or
by addition of a derivatization reagent, the derivatization reagent being selected from an acid or a base and capable of converting the active ingredient to a base or acid addition salt which is more sparingly soluble in the composition than the free active ingredient;
c) the composition is then cooled and solidified.

2. Process according to Claim 1, wherein the alkylcellulose is selected from methylcellulose and ethylcellulose, the hydroxyalkylcellulose is selected from hydroxypropylcellulose, the hydroxyalkylalkylcelluose is selected from hydroxypropylmethylcellulose, the cellulose ester is selected from cellulose phthalates, cellulose acetate phthalate, hydroxypropylmethylcellulose phthalate and hydroxypropylmethylcellulose acetate succinate.

3. Process according to Claim 1, wherein the thermoplastic polymer is selected from polyvinylpyrrolidone having Fikentscher K values of from 12 to 100, and copolymers of from 30 to 70% by weight of N-vinylpyrrolidone and from 70 to 30% by weight of vinyl acetate.

4. Process according to any of the preceding claims, wherein the non-solvent is selected from water, alcohols, polyols and lipids.

5. Process according to any of the preceding claims, wherein the composition comprises at least one plasticizer.

6. Process according to Claim 1, in which
a) a powdered mixture of the polymer and of the active ingredient is introduced into an elongated extruder housing at an entrance end;
b) the mixture is heated in the extruder housing in order to obtain a melt;
c) the melt is moved through the extruder housing to an exit end of the extruder housing;
d) a sufficient counter-pressure is generated in the extruder housing so that the melt exits from the exit end of the extruder housing as a coherent extrudate; and
e) crystallization of the active ingredient is initiated, by introducing the non-solvent or the derivatization reagent into the extruder housing at a point of the extruder housing located towards the exit end and mixing with the melt.

## Revendications

1. Procédé de fabrication de formes posologiques qui comprennent une dispersion solide d'un principe actif microcristallin, dans lequel
a) on fait fondre un polymère thermoplastique ayant une température de transition vitreuse Tg d'au moins 40°C, et on dissout jusqu'à homogénéité dans la masse fondue un principe actif qui se dissout dans 100 ml d'eau à 22°C en une quantité inférieure à 5 g,
le polymère thermoplastique étant choisi parmi la polyvinylpyrrolidone, les copolymères de N-vinylpyrrolidone et d'acétate de vinyle et/ou de propionate de vinyle, les copolymères d'acétate de vinyle et d'acide crotonique, le poly(acétate de vinyle) partiellement saponifié, le poly(alcool vinylique), les poly(acrylates d'hydroxyalkyle), les poly(méthacrylates d'hydroxyalkyle), les polyacrylates et les polyméthacrylates, les copolymères de méthacrylate de méthyle et d'acide acrylique, les polyéthylèneglycols, les alkylcelluloses, les hydroxyalkylcelluloses, les hydroxyalkyl-alkylcelluloses et les esters de cellulose ;
b) on amorce dans la masse obtenue une cristallisation du principe actif
par addition d'un non-solvant, le non-solvant étant un composé dans lequel moins de 1 g du principe actif est soluble dans 100 ml du non-solvant à la température de l'addition, ou
par addition d'un réactif de dérivatisation, le réactif de dérivatisation étant choisi parmi un acide ou une base et étant à même de convertir le principe actif en un sel d'addition avec un acide ou avec une base, qui dans la masse est moins soluble que le principe actif libre ;
c) puis on refroidit et solidifie la masse.

2. Procédé selon la revendication 1, dans lequel l'alkylcellulose est choisie parmi la méthylcellulose et l'éthylcellulose, l'hydroxyalkylcellulose parmi l'hydroxypropylcellulose, l'hydroxyalkyl-alkylcellulose parmi l'hydroxypropylméthylcellulose, l'ester de cellulose parmi les phtalates de cellulose, l'acétophtalate de cellulose, le phtalate d'hydroxypropylméthylcellulose et l'acétosuccinate d'hydroxypropylméthylcellulose.

3. Procédé selon la revendication 1, dans lequel le polymère thermoplastique est choisi parmi une polyvinylpyrrolidone ayant une valeur K selon Fikentscher de 12 à 100, et les copolymères de 30 à 70 % en poids de N-vinylpyrrolidone et de 70 à 30 % en poids d'acétate de vinyle.

4. Procédé selon l'une des revendications précédentes, dans lequel le non-solvant est choisi parmi l'eau, les alcools, les polyols et les lipides.

5. Procédé selon l'une des revendications précédentes, dans lequel la masse comprend au moins un plastifiant.

6. Procédé selon la revendication 1, dans lequel
a) on introduit, par une extrémité d'entrée, un mélange pulvérulent du polymère et du principe actif dans un fourreau d'extrudeuse longitudinal ;
b) on chauffe le mélange dans le fourreau de l'extrudeuse, pour obtenir une masse fondue ;
c) on déplace la masse fondue à travers le fourreau de l'extrudeuse jusqu'à une extrémité de sortie du fourreau de l'extrudeuse ;
d) on produit dans le fourreau de l'extrudeuse une contre-pression suffisante pour que la masse fondue sorte de l'extrémité de sortie du fourreau de l'extrudeuse sous forme d'un extrudat continu ; et
e) on amorce une cristallisation du principe actif en introduisant dans le fourreau de l'extrudeuse, en un point du fourreau de l'extrudeuse disposé vers l'extrémité de sortie, le non-solvant ou le réactif de dérivatisation, et on le mélange à la masse fondue.
